# EUROPEAN PATENT SPECIFICATION

(11) **EP 1 439 220 B1**
(45) Date of publication and mention of the grant of the patent: **22.08.2007**
(21) Application number: 03425018.3
(22) Date of filing: 16.01.2003
(51) Int. Cl.: C12N 9/10, C12P 19/02, C12P 19/38, C12P 19/40, C12N 11/18, C12N 11/08

(54) **Immobilized biocatalysts usable for the manufacture of natural nucleosides and modified analogues through enzymatic transglycosylation reactions**
Immobilisierte Biokatalysatoren für die Herstellung von natürlichen Nukleosiden und modifizierten Analogen durch enzymatische Transglykosylierungsreaktionen
Biocatalyseurs immobilisés pour la production des nucléosides naturels et des analogues modifiés par des réactions enzymatiques de transglycolysation

(43) Date of publication of application: 21.07.2004
(73) Proprietor: Adorkem Technology SpA, 24062 Costa Volpino (Bergamo) (IT)
(72) Inventor: Tonon, Giancarlo, 20139 Milano (IT); Capra, Emanuele, 26900 Lodi (IT); Orsini, Gaetano, 21013 Gallarate (Varese) (IT); Zuffi, Gabriele, 20026 Novate Milanese (Milano) (IT)
(74) Representative: Pistolesi, Roberto

(56) References cited:
- EP-A- 0 346 865
- EP-A- 0 897 014
- WO-A-03/008619
- US-A- 5 314 810
- MAHMOUDIAN M: "BIOCATALYTIC PRODUCTION OF CHIRAL PHARMACEUTICAL INTERMEDIATES" BIOCATALYSIS AND BIOTRANSFORMATION, HARWOOD ACADEMIC PUBL., BASEL, CH, vol. 18, no. 2, 2000, pages 105-118, XP009002518 ISSN: 1024-2422
- ROSSI C A ET AL: "REVERSIBLE IMMOBILIZATION OF GUANINE DEAMINASE BY COVALENT CHROMATOGRAPHY" JOURNAL OF MOLECULAR CATALYSIS, vol. 2, no. 3, 1977, pages 163-170, XP009009816 ISSN: 0304-5102
- BARAI V N ET AL: "A universal biocatalyst for the preparation of base- and sugar-modified nucleosides via an enzymatic transglycosylation" HELVETICA CHIMICA ACTA 2002 SWITZERLAND, vol. 85, no. 7, 2002, pages 1901-1908, XP002239460 ISSN: 0018-019X
- ESIPOV ROMAN S ET AL: "Overexpression of Escherichia coli genes encoding nucleoside phosphorylases in the pET/Bl21 (DE3) system yields active recombinant enzymes." PROTEIN EXPRESSION AND PURIFICATION, vol. 24, no. 1, February 2002 (2002-02), pages 56-60, XP002239461 February, 2002 ISSN: 1046-5928
- SPOLDI E ET AL: "Recombinant bacterial cells as efficient biocatalysts for the production of nucleosides." NUCLEOSIDES, NUCLEOTIDES & NUCLEIC ACIDS. UNITED STATES 2001 APR-JUL, vol. 20, no. 4-7, April 2001 (2001-04), pages 977-979, XP009009766 ISSN: 1525-7770
- GAEVAIA L V ET AL: "[The use of immobilized enzymes for the synthesis of nucleosides and nucleotides including the ones labeled with radioactive isotopes]" PRIKLADNAIA BIOKHIMIIA I MIKROBIOLOGIIA. USSR 1987 MAY-JUN, vol. 23, no. 3, May 1987 (1987-05), pages 309-316, XP009009807 ISSN: 0555-1099

## Description

The present invention relates to the preparation of novel biocatalysts usable in industrial processes for the production of natural nucleosides and of modified analogues which can be used in the pharmaceutical field as intermediates or as finished products, in the field of anti-tumour and antiviral drugs, as well as in the preparation of intermediates for the synthesis of oligonucleotides of therapeutic interest.

Natural nucleosides, nucleoside analogue compounds and their derivatives, modified variously either with respect to the ribofuranosyl group or with respect to the heterocyclic base bound to the sugar, can be produced by conventional chemical synthesis methods which, particularly for purine derivatives, present problems relating to unsatisfactory yields and/or incomplete stereo-selectivity of the correct anomeric position and/or to the need to use reactions for the protection/deprotection of labile chemical groups and/or to use complex purification methods.

Alternative approaches to the preparation of nucleosides and analogues, which are based on the use of enzymes and which may overcome the problems of preparation by chemical synthesis, are known from scientific and patent literature.

In this field, a class of enzymes known as nucleoside phosphorylases is particularly interesting; these catalyze both phosphorolysis reactions (consisting of the detachment of the sugar from a nucleoside to give the corresponding sugar-1-phosphate) and the reverse reactions (consisting of the condensation of the sugar-1-phosphate to a purine or pyrimidine base or to a nitrated heterocyclic acceptor) to form a new nucleoside. The combination of the two reactions, catalyzed by two phosphorylases with different substrate specificities, enables a so-called transglycosylation reaction to be performed; in the presence of phosphate ions, this consists of the transfer of the ribofuranosyl or modified ribofuranosyl group from a suitable nucleoside which has the function of a sugar donor to a suitable heterocyclic base which behaves as a sugar acceptor, giving rise to the formation of a new nucleoside in accordance with a reaction equilibrium which depends on the chemical/physical characteristics of the various substrates, on their relative concentrations, and on the operative conditions used, for example, such as pH and temperature. [Otto J.L., Werkman C.H. Coupled nucleoside phosphorylase reactions in Escherichia coli. Arch. Biochem. Biophys. 69, 264-276, 1957; Doskocil J., Holy A. Specificity of purine nucleoside phosphorylase from Escherichia coli. Collect. Czech. Chem. Commun. 42, 370-382, 1977; Utagawa T. et al., Mechanism of purine arabinoside synthesis by bacterial transarabinosylation reactions. 49 (8), 2425-2430, 1985].

In particular, the enzymes uridine phosphorylase or UdP (E.C. 2.4.2.3) and purine nucleoside phosphorylase or PNP (E.C. 2.4.2.1.) have been used in combination for the preparation of natural or modified nucleosides in transglycosylation reactions which use various pyrimidine nucleosides such as, for example, uridine, 2'-deoxyuridine, 2'-3',-dideoxyuridine, or arabinofuranosyl uracil as sugar donors and a variety both of natural or modified purine bases and. of nitrated heterocyclic compounds, acting as sugar acceptors. [Hutchinson D.W. New approaches to the synthesis of antiviral nucleosides. Trends Biotechnol. 8, 348-353, 1990; Hanrahan J.R., Hutchinson D.W. The enzymatic synthesis of antiviral agents. J.Biotechnol. 23, 193-2120, 1992].

The isolation and the study of UdP and PNP from different species has shown that enzymes derived from prokaryotic microorganisms have a broader substrate specificity and greater thermal stability in comparison, for example, with the corresponding enzymes isolated from mammals, and are therefore preferable as catalysts for industrial use [Mao C. et al., The crystal structure of E.coli purine nucleoside phosphorylase. A comparison with the human enzyme reveals a conserved topology. Structure 5, 1373-1383, 1997; Browska A. et al., Properties of purine nucleoside phosphorylase of mammalian and bacterial origin. Z.Naturforsch. 45c, 59-70, 1989].

UdP and PNP isolated from prokaryotic microorganisms in the form of soluble enzymes have been used as catalysts in transglycosylation reactions in homogeneous phase; however, this approach is not very efficient and is generally limited to the laboratory scale since it requires the use of the purified enzymes which cannot be reused for successive reaction cycles, it does not permit operation at temperatures above 37-40°, and it requires particularly long reaction times [Krenitsky T.A. et al., Enzymatic synthesis of purine arabinonucleosides. EP-0002192, 1979; Krenitsky T.A. et al. Purine nucleoside synthesis, an efficient method employing nucleoside phosphorylase. Biochemistry 20, 3615-3621, 1981; Krenitski T.A. et al. An enzymic synthesis of purine D-arabinonucleosides. Carbohydrate Res. 97, 139-146, 1981; Krenitsky T.A. et al. Imidazo [4,5-c] pyridines (3-deaza purines) and their nucleosides as immunosuppressive and 'anti-inflammatory agents. J.Med.Chem. 29, 138-143, 1986].

The discovery that the enzymes UdP and PNP are present simultaneously in the cytoplasm of numerous wild-type strains of microorganisms belonging to various genera, in proportions compatible with the function of catalyzing transglycosylation reactions, has suggested their direct use, that is, in the form of whole cell preparations, as biocatalysts in the preparation of nucleosides and of modified analogues. The advantages of this approach are represented by the possibility of avoiding the extraction and purification of UdP and PNP, as well as by the stability of the cytoplasmic enzymatic activity which enables the transglycosylation reactions to be carried out at temperatures of up to about 60°C, thus favoring the solubilization of the substrates and increasing the rate of reaction.

Many works have already documented the applicability of transglycosylation reactions catalyzed by whole cells of microorganisms belonging to various species such as, for example *Enterobacter aerogenes, Erwinia herbicola, Brevibacterium acetylicum, Escherichia coli,* both at laboratory level and on a production scale.

[Utagawa et al., Properties of nucleoside phosphorylase from Enterobacter aerogenes. Agric.Biol.Chem. 49, 3239-3246, 1985; Utagawa T. Enzymatic preparation of nucleoside antibiotics. J.Molec.Catalysis B: Enzymatic 6 (3-4), 215-222, 1999; Cotticelli G. et al., Production of nucleosides by large-scale bioconversion. Nucleosides Nucleotides 18(4-5), 1135-1136, 1999;

Yokozeki K, Tsuji T. A novel enzymatic method for the production of purine-2'-deoxynucleosides. J. Molec. Catalysis B: Enzymatic, 10, 207-213, 2000].

The use of whole bacterial cells, enabling the reactions to be carried out in heterogeneous phase, also potentially enables the cells to be recovered for recycling in successive reactions, although it is necessary to take into account the possibility of contamination resulting from more or less extensive cell lysis and the fact that the recovery of the cells complicates the production process, requiring further filtration and/or ultrafiltration or centrifuging steps. Approaches have also been described in which the recycling of the cells used as biocatalysts was performed with the use of cells pre-aggregated with glutaraldehyde [Zinchenko A.I. et al., Synthesis of 9-(β-D-arabinofuranosyl) guanine using whole cells of Escherichia coli. Appl. Microbiol. Biotechnol. 32, 658-661, 1990; Barai V.N. et al., A universal biocatalyst for the preparation of base- and sugar-modified nucleosides via an enzymatic transglycosylation. Helv.Chim.Acta 85, 1901-1907, 2002], by encapsulation of the cells in hydrophilic matrices [Votruba I. et al., Synthesis of 2-deoxy-β-D-ribonucleosides and 2-3-dideoxy-β-D-pentofuranosides on immobilized bacterial cells. Collect.Czech.Chem.Commun. 59, 2302-2330, 1994], or by incorporation of the cells in hydrophobic polymer resins [Yokozeki K. et al., Production of adenine arabinoside by gel-entrapped cells of Enterobacter aerogenes in water-organic cosolvent system. Eur. J. Appl. Microbiol. Biotechnol. 14, 225-231, 1982].

Finally, the construction of genetically modified bacterial strains which can express high levels of the recombinant forms of the enzymes UdP and PNP, both separately in different cells and simultaneously in the same cell, [Bestetti G. et al., Recombinant bacterial strains for the production of natural nucleosides and modified analogues thereof. WO 00/39307, 1999] has enabled microorganisms to be obtained which can advantageously be used both for the production of UdP and PNP [Esipov R.S. et al., Overexpression of Escherichia coli genes encoding nucleoside phosphorylases in the pET/B121 (DE3) system yields active recombinant enzymes. Protein Express. Purif. 24, 56-60, 2002] and for the use of the whole cells as biocatalysts in the production of natural nucleosides and analogues by transglycosylation reactions [Spoldi E. et al., Recombinant bacterial cells as efficient biocatalysts for the production of nucleosides. Nucleosides Nucleotides 20 (4-7), 977-979, 2001].

However, it is generally accepted that enzymes immobilized on a solid substrate constitute the physical form which is most advantageous for producing an industrial biocatalyst since they enable the reactions to be carried out in heterogeneous phase, minimizing or eliminating the problem of contamination by biological material, they can be prepared in a physical form that is easily separable from the reaction mixture for possible reuse, they facilitate the process for the purification of the reaction mixture, and they simplify the production process since they are compatible with any reactor configuration for performing both discontinuous and continuous reactions. Since the enzymatically active forms of UdP and PNP are constituted by homo-hexameric complexes, their immobilization on a solid substrate presents a series of problems because the immobilization process must be compatible with the maintenance of the active oligomeric configuration [Pugmire M.J., Ealick S.E. Structural analyses reveal two distinct families of nucleoside phosphorylase. Biochem.J. 361, 1-25, 2002]. It has been reported that, in comparison with the methods for immobilization by the formation of covalent bonds, methods of immobilization by non-covalent adsorption typically disturb the structural characteristics of the enzymes much less and consequently facilitate the maintenance of the immobilized enzyme in a configuration comparable to the active natural configuration that is present in solution [Bailey J.E., Ollis D.F. (a cura di). Biochemical engineering fundamentals. 2nd edition, McGraw-Hill, New York, 1986, pag. 157].

In line with these indications, the only form of co-immobilization of UdP and PNP enzymes of prokaryotic origin that is known up to now is based on adsorption by non-covalent interactions of purified preparations of the enzymes on DEAE weak basic cellulose resin. However, this approach has limitations from the point of view of industrial application since the preparation of the catalyst requires the separate purification of the two enzymes prior to the process of adsorption on resin to eliminate the presence of other extraneous enzymes which would be co-adsorbed and would interfere in the subsequent transglycosylation reaction; moreover, the reuse of the immobilized enzyme is limited to a few reaction cycles [Averett D.R. et al. 6-Methoxypurine arabinoside as a selective and potent inhibitor of varicella-zoster virus. Antimicrob. Agents Chemother. 35 (5), 851-857, 1991; Mahmoudian M. Biocatalytic production of chiral pharmaceutical intermediates. Biocatalysis Biotransform. 18, 105-118, 2000] .

It has now unexpectedly been found, and constitutes the principal subject of the present invention, that the co-immobilization of both of the enzymes UdP and PNP by the formation of covalent bonds with a solid matrix functionalized with epoxy groups enables biocatalysts with high specific activity, excellent thermal stability, and compatibility with high solvent concentrations to be produced. Since these novel immobilized catalysts are easily separable from the reaction mixture and are reusable for numerous successive reaction cycles, they are particularly suitable for industrial use and solve the problems which have been associated with the industrial production of nucleosides by transglycosylation reactions up to now.

A further subject of the present invention is represented by the possibility of using, in the co-immobilization process, not only the isolated enzymes, but also the crude soluble fractions of the enzymes UdP and PNP produced from cell cultures (where this expression means the fraction which, after cell lysis and centrifuging, is recovered as supernatant liquid - constituted by the lysis buffer containing the cell components, including the enzymes which are soluble in the buffer - which is separated from the insoluble pellet constituted by cell-wall residues and non-lysed or only partially lysed cells); it has in fact unexpectedly been found that all of the contaminating enzymatic activities can be completely inactivated prior to the co-immobilization process by subjecting the cells of the microorganisms producing the enzymes to a heat treatment at a temperature of 50-65°C and preferably at a temperature of 60°C, carried out for a period variable from 10 minutes to 120 minutes and preferably for a period of 30 minutes, without the heat treatment prejudicing the enzymatic activity of UdP and PNP.

According to one embodiment of the present invention, the best results for the co-immobilization of UdP and

PNP have been achieved by the formation of covalent bonds with a solid matrix functionalized with oxyranic groups (epoxy groups) by means of a reaction which involves mainly the lateral amino groups of the lysine residues that are present on the polypeptide chains of the enzymes, in accordance with a process which does not require drastic reaction conditions such as, for example, exposure to high temperatures or wide pH ranges, or the use of high concentrations of chaotropic agents. It has also been found advantageous to use, as immobilization substrates, commercially available epoxy resins for which some technological characteristics which are important for industrial use, such as, for example, particle size, their high density and porosity, their resistance to compression, their resistance to microbial attack, etc., are known and guaranteed.

According to a further embodiment of the present invention, the enzymes UdP and PNP are co-immobilized by the formation of covalent bonds on solid substrates such as, for example, agarose gel, silica gel, polymers based on methacrylamide-bisacrylamide or polymethacrylate polymers functionalized with epoxy groups. Amongst the various solid substrates which are commercially available and which are usable for the co-immobilization of UdP and PNP as described in the present invention, the following materials, amongst others, may be mentioned: Epoxy-activated Sepharose® ; Fractogel® TSK AF-Epoxy; Eupergit® C and Eupergit® C250L; Sepabeads® EC-EP/M and Sepabeads® EC-EP.

Although all of the materials given above can be used as substrates for the co-immobilization of the enzymes UdP and PNP by the formation of covalent bonds, the solid substrates constituted by methacrylic polymers functionalized with oxyranic groups (epoxy groups) which are present at a concentration of no less than 50 µmoles/gram of moist resin and preferably at a concentration of about 100 µmoles/gram of moist resin, have been found particularly suitable for the preparation of a catalyst usable industrially for the production of nucleosides. The resin Sepabeads EC-EP/M or equivalent resins that are characterized by having a particle size variable between about 200 and 600 microns and a concentration of active epoxy groups equal to or greater than 100 µmoles/gram of moist resin are preferably used for the co-immobilization of the enzymes UdP and PNP in accordance with the operative methods described in the present invention.

These resins are obtainable commercially in preactivated form, that is, carrying the epoxy groups in a state of high reactivity capable of rapidly forming covalent bonds by reacting preferably with the ε-amino group of the lysine residues that are present in the polypeptide chains of the enzymes to be immobilized, in accordance with the following reaction scheme:

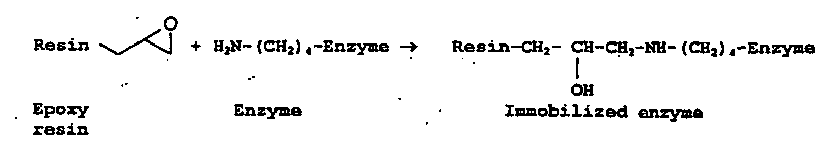

The. co-immobilization of the enzymes UdP and PNP on Sepabeads epoxy resins or on equivalent resins was investigated by stirring the crude enzymatic mixture with the immobilization substrate in the presence of a buffer with a pH of about 7-7.5 for a period of up to 72 hours.

The final cell lysis and co-immobilization conditions were established by investigating the following operative parameters (the optimal conditions selected for each of the parameters investigated are given in brackets):
a) lysis of the cell biomass and immobilization in 0.5 M, 1M, and 1.5 M phosphate buffer (condition selected: 1M phosphate buffer)
b) immobilization temperature about 20°C (ambient temperature) and 50°C (condition selected: ambient temperature)
c) contact time between the mixture of the crude cell lysates and the resin of 12 hours, 24 hours, 48 hours and 72 hours (condition selected: 48 hours)
d) ratio between UdP enzymatic activity and PNP enzymatic activity in the mixture of the crude cell lysates: UdP/PNP = 0.5; UdP/PNP = 1; UdP/PNP = 2; UdP/PNP = 3 (condition selected: UdP/PNP enzymatic-activity ratio = 1)
e) ratio between transglycosylation catalytic activity (expressed in units · ml⁻¹ as defined in Example 3c) of the mixture of the crude cell lysates and the quantity of resin: 90 units per 2 grams of moist resin; 90 units per 3 grams of moist resin; 90 units per 4 grams of moist resin (condition selected: approximately 90 units/3 grams of moist resin, corresponding to about 30 units/gram of moist resin)
f) concentration of transglycosylation catalytic activity (expressed in units · ml⁻¹ as defined in Example 3c) in the mixture of crude cell lysates of 2 units · ml⁻¹, 6 units · ml⁻¹, and 10 units ·ml⁻¹ (condition selected: about 6 units · ml⁻¹)
g) post-immobilization treatment of the immobilized catalyst; no further treatment, conditioning in pH 8.5 phosphate buffer, conditioning in phosphate-glycine buffer (condition selected: no further treatment).

On the basis of the results obtained in these tests, the preparation of the immobilized catalyst, in accordance with one of the preferred applications of the present invention, comprises the following operative steps:
a) The enzymes UdP and PNP are produced by separate fermentation of recombinant strains of *Escherichia coli* transformed with the gene udp of *E.coli* encoding for the enzyme UdP and with the gene *deoD* of *E.coli* encoding for the enzyme PNP, respectively. Recombinant strains usable for the production of the enzymes UdP and PNP are similar, for example, respectively, to the strain DH5α/pGM708 (referred to as NP23/3 in the present patent) and to the strain DH5α/pGM707 (referred to as NP24/3 in the present patent) which are described in WO 00/39307, which is incorporated herein by reference; the characteristics of these strains are given in Table 1. In accordance with the instructions recently issued by the international regulatory authorities and in order to eliminate any risk of contamination by causative agents of bovine spongiform encephalopathy, the recombinant strains NP23/3 and NP24/3 were recloned without the use of materials of animal origin; the preparation and preservation of the cell banks and the fermentation of the recombinant strains were similarly carried out without the use of materials of animal origin [EMEA. Note for guidance on minimising the risk of transmitting animal spongiform encephalopathy agents via human and veterinary medicinal products. 2001].

**Table 1. Characteristics of the recombinant strains of Escherichia coli that are producers of UdP and PNP**

| **Strain** | **Protein cloned** | **Selection marker** |
|---|---|---|
| NP23/3 | Uridine phosphorylase (UdP) | Tetracycline |
| NP24/3 | Purine nucleoside phosphorylase (PNP) | Tetracycline |

b) After thermal treatment at 60° for 30 minutes in order to inactivate the contaminating enzymatic activities, the biomasses obtained in the two fermentations are subjected to mechanical lysis (or alternatively to lysis by sonication and to chemical and/or enzymatic lysis) and centrifuged to separate the crude soluble fractions; the mixture of the two soluble fractions is then used directly for the immobilization without further purification steps.
c) The crude cell lysates containing UdP and PNP are mixed so as to have UdP : PNP enzymatic-activity ratios variable from 1 : 0.5 to 1 : 3 (and preferably a ratio of about 1: 1) and are put in contact with the immobilization substrate.
d) The immobilization substrate is constituted by an acrylic resin functionalized with epoxy groups, and preferably by the resin Sepabeads EC-EP/M, and is used in a proportion of about 2-3 kg of moist resin to immobilize 10 litres of mixture of crude cell lysates having a transglycosylation catalytic activity of about 6 units · ml⁻¹.

As an alternative to the method given above, the crude cell lysates containing the enzymes UdP and PNP may be immobilized separately by using a method similar to the method described for the co-immobilization of the two enzymes and then mixed for use as catalysts of the transglycosylation reactions.

The use of recombinant strains for the production of the enzymes UdP and PNP enables large cell biomass quantities to be obtained and enables cell lysates to be extracted with a high enzyme titre, as shown in Tables 2 and 3 below.

**Table 2. Production of UdP by fermentation of the recombinant strain NP23/3**

| **Fermentation** | **Cell Biomass*** Grams/litre | **UdP Activity**** Units/litre |
|---|---|---|
| 1 | 162 | 476300 |
| 2 | 160 | 376300 |
| 3 | 185 | 511400 |
| 4 | 160 | 480000 |

| | | |
|---|---|---|
| ** The cell biomass is given as moist weight* | | |
| *** The enzymatic activity is measured on the cell lysate and relates to the phosphorolysis of uridine reaction* | | |

**Table 3. Production of PNP by fermentation of the recombinant strain NP24/3**

| **Fermentation** | **Cell biomass*** Grams/litre | **UdP Activity**** Units/litre |
|---|---|---|
| 1 | 185 | 111000 |
| 2 | 166 | 87150 |
| 3 | 165 | 111400 |
| 4 | 168 | 100800 |

| | | |
|---|---|---|
| ** The cell biomass is given as moist weight* | | |
| *** The enzymatic activity is measured on the cell lysate and relates to the phosphorolysis of inosine reaction* | | |

In the co-immobilization method, the crude cell lysates containing UdP and PNP can be mixed in different proportions so as to obtain a catalyst which is optimized for each transglycosylation reaction of interest. For example, it has been found that, by starting with lysate mixtures with UdP: PNP enzymatic-activity ratios variable from 2 : 1 to 1 : 2, immobilized catalysts are obtained that are optimal for use in most of the glycosylation reactions which use ribofuranosyl uracil, 2'-deoxyribofuranosyl uracil, 2'-3'-deoxyribofuranosyl uracil and arabinofuranosyl uracil as sugar donors.

In the operative conditions described, a catalyst which has the analytical characteristics given in Table 4 is obtained with a yield of the co-immobilizations process, calculated as the transglycosylation catalytic activity, of about 50-70%.

**Table 4. Analytical characteristics of the catalyst obtained by co-immobilization of the enzymes UdP and PNP by the formation of covalent bonds on resin functionalized with epoxy groups**

| **Parameters** | **Characteristics*** |
|---|---|
| Appearance | White-brown granules |
| Particle size | 200-600 micrometres |
| Water | 40-60% |
| pH | 6-8 |
| Enzymatic Activity** | 10-20 units/gram |

| | |
|---|---|
| ** Relating to the filtered moist resin* | |
| *** Transglycosylation catalytic activity determined as described in Example 3c.* | |

The immobilized enzyme preparation is preserved at 4°C as moist resin in 100 mM potassium phosphate buffer - 20% isopropanol - pH 7 - 500 ppm ethyl p-hydroxy benzoate. In these conditions, complete maintenance of the transglycosylation catalytic activity has been confirmed in a stability study carried out for up to 6 months.

The method of the present invention for the covalent co-immobilization of the enzymes UdP and PNP permits the preparation of a novel form of immobilized catalyst which is stable up to temperatures of 60 - 70°C, is compatible with the presence of a high concentration of water-miscible solvents, such as, for example, alcohols, ethylene and polyethylene glycols, dimethyl sulphoxide, and tetrahydrofuran, and which maintains a good enzymatic activity within the pH range of from 6 to 9.

According to a further application of the present invention, the enzymes UdP and PNP, immobilized on a solid substrate functionalized with epoxy groups by the formation of covalent bonds, are advantageously usable for the industrial preparation of natural nucleosides and analogues modified by transglycosylation reactions starting with a sugar-donor nucleoside and a sugar-acceptor base. The immobilized catalyst of the present invention has characteristics of enzymatic efficiency and stability and mechanical properties such as to enable transglycosylation reactions to be carried out at about 50-60°C, to enable the reaction mixture to be separated easily form the catalyst upon completion of the reaction (for example, by direct filtration on a porous partition welded to the reaction vessel) and enables the same catalyst to be reused for successive reaction cycles as described in greater detail in the examples given below.

Thus, for example, in a cycle of 12 successive reactions carried out at 60°C for 2.5 hours for the preparation of β-D-arabinofuranosyl 2,6 diaminopurine (Ara-DAMP) in which, in each reaction, the resin recovered in the previous reaction was reused as catalyst, it was found that the resin recovered after the last cycle (for a total period of operative use at 60°c of 30 hours) had retained more than 85% of its initial catalytic activity. The stability of the catalyst was further investigated at 60°C, 50°C and 40°C for continuous operative periods of up to 240 hours (10 days); the results obtained indicated a mean loss of catalytic efficiency of 8% per 24 hours of operative use at a temperature of 60°C, and of 5% per 24 hours of operative use at a temperature of 50°C whereas, at a temperature of 40°C, the mean loss of catalytic efficiency per 24 hours of operative use was less than 0.5%.

According to a further application of the present invention, the immobilized catalyst is also usable in reactions which use substrates that are poorly soluble in aqueous buffers, as is the case with the preparation of fludarabine desphosphate which is described in one of the examples given below in which the presence of 40% of dimethyl sulphoxide in the reaction mixture, which was heated to a temperature of 60°C, did not modify the activity of the immobilized catalyst.

According to a further application of the present invention, the immobilized catalyst can be used in transglycosylation reactions carried out in a continuous reactor. The reactor may advantageously be constituted by a thermostatically controlled column; the reaction mixture, containing the sugar-donor nucleoside, the acceptor base, and the phosphate buffer as described in one of the examples given below, which relates to the preparation of 2'-deoxyadenosine, is passed through the column by gravity or, preferably, by means of a calibrated-flow pump. By adjusting the flow of the reaction mixture through the catalyst bed in relation to the rate of the transglycosylation reaction (in the conditions used for the production of 2'-deoxyadenosine, the optimal flow was 1/5 of the column volume/minute) the enzymatic reaction reaches equilibrium in the time taken to pass through the catalyst and the eluate of the column can be used directly for the purification of the new nucleoside formed in the reaction.

The following examples are intended to illustrate the present invention without constituting a limitation of its field of application.

### Example No. 1.

### Fermentation of recombinant strains which are producers of UdP and PNP

The recombinant strains NP23/3 and NP24/3 which express the enzymes UdP and PNP, respectively, were fermented separately in fed-batch conditions with the use of fermenters with a useful fermentation volume of 10 litres, containing 8 litres of culture medium at pH 6.8-7.0 having the following composition (per litre): 13.3 g KH₂PO₄; 4.0 g (NH₄)₂HPO₄; 1.7 g citric acid, 1.25 g soitone; 2.5 g glycerol; 0.125 g yeast extract; 1.5 g MgSO₄.7H₂O; 0.08 g CaCl₂; 0.08 g FeSO₄.7H₂O; 0.02 g MnSO₄.H₂O; 0.03 g ZnSO₄.7H₂O; 0.003 g H₃BO₃; 0.006 g CuSO₄.5H₂O; 0.008 g CoCl₂.6H₂O; 0.004 g NaMoO₄.2H₂O; 0.010 g thiamine.HCl, 0.0125 g tetracycline. The fermenter was inoculated at an initial OD₆₀₀ value of 0.4 with about 300 ml of bacterial suspension previously grown for about 20 hours at 30°C. The fermentation was carried out with the use of the following operative parameters: temperature 30°C; air flow of 1 volume/volume of bouillon culture/minute; initial stirring of 150 revolutions/minute increased automatically to keep the pO₂ value at 20% of the saturation concentration for 10 hours (batch phase) and then at 10% of the saturation concentration; pH kept at 7.0 ±0.1 by automatic addition of a solution of 12.5% NH₄OH or of a 25% solution of H₃PO₄. During the fed-batch phase (from 10 hours after the start of fermentation and up to about 50 hours), 2 litres of a solution having the following composition (per litre) was added automatically to the fermenter: 400 g glycerol; 200 g soitone; 20 g yeast extract; 3 g MgSO₄.7H₂O; 0.0125 g tetracycline. Upon completion of the fermentation (which was completed in about 51-52 hours) the cell biomass was collected by centrifuging, washed in 30 mM phosphate buffer - pH 7, recentrifuged, and kept as moist cell biomass at a temperature of - 20°C.

### Example No. 2

### Cell lysis

a) 400 grams of moist cell paste, separated by centrifuging or by microfiltration from a bouillon culture of the recombinant strain NP23/3 which expresses the enzyme UdP, was resuspended to give a volume of 1.5 litres with 1M phosphate buffer - pH 7.5 and heated with stirring to 56-60°C for 30 minutes. After cooling to about 4°C, the suspension was lysed by means of three consecutive steps at a pressure of about 600 bar in a Manton-Gaulin mechanical homogenizer with the temperature kept at about 10°C. The suspension was clarified by centrifuging at 12,000 x g, giving about 2 litres of solution containing the total soluble proteins.
b) 1600 grams of moist cell paste, separated by centrifuging from a bouillon.culture of the recombinant strain NP24/3 which expresses the enzyme PNP, was resuspended to give a volume of 8 litres with 1M phosphate buffer - pH 7.5 and heated with stirring to 56-60°C for 30 minutes. After cooling to about 4°C, the suspension was lysed by means of three consecutive steps at a pressure of about 600 bar in a Manton-Gaulin mechanical homogenizer with the temperature kept at about 10°C. The suspension was clarified by centrifuging at 12,000 x g, giving about 8 litres of solution containing the total soluble proteins.

### Example No. 3.

### Determination of enzymatic activities

a) Determination of the enzymatic activity of UdP.
   A measured volume (from 100 to 200 microlitres) of centrifuged lysate, diluted 1:100 v/v, extracted from the cell paste which expressed UdP, was added to 800 microlitres of a 75 mM solution of uridine in 100 mM potassium phosphate buffer - pH 7, preincubated at 30°C. After 5 minutes, the uridine → uracil phosphorolysis reaction was stopped by the addition of 1 ml 1N HCl. An aliquot of the reaction mixture was analyzed by high pressure liquid chromatography (HPLC) with the use of a 250 x 4.6 mm, 5 µm spheres, C-18 Capcell-Pack column (Shisheido) eluted with a 30 mM solution of monobasic ammonium phosphate - pH 4.5 - 9% methanol. The enzymatic activity of the cell lysate was expressed as units · ml⁻¹ (µmoles of uracil · min⁻¹ · ml⁻¹) and was calculated relative to a standard uracil solution eluted in the same conditions.
b) Determination of PNP enzymatic activity.
   A measured volume (variable from 100 to 200 microlitres) of centrifuged lysate diluted 1:100 v/v, extracted from the cell paste of the strain which expresses PNP, was added to 800 microlitres of a 62.5 mM solution of inosine in 100 mM potassium phosphate buffer - pH 7, preincubated at 30°C. After 10 minutes, the inosine → hypoxanthine phosphorolysis reaction was stopped by the addition of 1 ml of 1N HCl. An aliquot of the reaction mixture was analyzed by high pressure liquid chromatography (HPLC) with the use of a 250 x 4.6mm, 5 µm spheres, C-18 Capcell-Pack column (Shisheido) eluted with a 30 mM solution of monobasic ammonium phosphate - pH 4.5 - 9% methanol. The enzymatic activity of the cell lysate was expressed as units · ml⁻¹ (µmoles of hypoxanthine · min⁻¹ · ml⁻¹) and was calculated relative to a standard hypoxanthine solution eluted in the same conditions.
c) Determination of transglycosylation catalytic activity.
   The transglycosylation catalytic activity of the mixture of cell lysates containing UdP and PNP or of the enzymes UdP and PNP co-immobilized on a solid substrate was determined in a transglycosylation reaction carried out on analytical scale in standardized conditions.
   250 µl of the mixture of cell lysates or 100 mg (moist weight) of immobilized UdP-PNP catalyst was added to 10 ml of a solution having the following composition: 40 mM β-D-arabinofuranosyl uracil (Ara-U), 40 mM adenine, 30 mM potassium phosphate buffer - pH 7, thermostatically controlled at 60°C. After 1.5 hours at 60°C, the reaction was stopped by diluting the mixture 1:50 and cooling in ice. The percentage of bioconversion of adenine to β-D-arabinofuranosyl adenine (Ara-A) was determined by analyzing an aliquot of the reaction mixture by high pressure liquid chromatography (HPLC) with the use of a 250 x 4.6 mm, 5 µm spheres, C-18 Capcell-Pack column (Shisheido), eluted with a 30 mM solution of monobasic ammonium phosphate, pH 4.5 - 9% methanol. The transglycosylation catalytic activity was expressed as units · ml⁻¹ (µmoles of Ara-A formed in 1.5 hours · ml⁻¹ of mixture of cell lysates) or in units · g⁻¹ of moist resin (µmoles of Ara-A formed in 1.5 hours · g⁻¹ of moist resin) and was calculated relative to a standard Ara-A solution eluted by HPLC in the same conditions.

### Example No. 4.

### Preparation of the catalyst by co-immobilization of UdP and PNP

The co-immobilization of the enzymes UdP and PNP was carried out starting with a mixture of the lysates prepared so as to have a UdP and PNP activity ratio variable within the range of from 1 : 0.5 to 1 : 4. In this example, immobilization starting with a mixture of lysates in which the UdP : PNP activity ratio was 1 : 1 is described.

2.6 kg (moist weight, containing about 40-50% of H₂O) of Sepabeads EC-EP/M epoxy resin (Resindion Srl, Milan) and 500 parts per million of p-hydroxymethyl benzoate was added to a mixture of 2 litres of crude cell lysate containing a UdP activity of about 500 units/ml and 8 litres of crude cell lysate containing a PNP activity of about 125 units/ml. The mixture was kept at ambient temperature with gentle stirring for 48 hours; the mixture was left to clear, the supernatant liquid was removed, the resin was filtered under vacuum in a Buchner filter and was washed, on the filter, with about 20 litres of deionized.water.

The immobilized catalyst was characterized by determining the transglycosylation activity and was kept at 4°C in 100 mM phosphate buffer - 20% propanol - 500 ppm ethyl p-hydroxybenzoate.

### Example No. 5

### Examples of preparation of nucleosides by the transglycosylation reaction catalyzed by the enzymes UdP and PNP co-immobilized by covalent bonds on a solid substrate

a) Preparation of Ara-A
   10 grams (moist weight) of immobilized catalyst with transglycosylation activity of about 12 units/gram, previously filtered from the preservation solution and washed on the filter with deionized water, was added to 150 ml of a solution kept thermostatically at 60°C and having the following composition:
   25 mM Ara-U
   25 mM adenine
   30 mM potassium phosphate buffer, pH 7.
   After 1 hour at 60°C, the catalyst was discharged (and was made to react in a new bioconversion reaction) and the filtrate was cooled in order to recover the precipitate, constituted by crude Ara-A, which was purified by crystallization from water.
   The bioconversion yield of the reaction was ≥ 70%. The purity of Ara-A after crystallization, determined by HPLC analysis, was greater than 98%.
b) Preparation of β-D-arabinofuranosyl adenine (Ara-DAMP)
   65 grams (moist weight of immobilized catalyst with transglycosylation activity of about 13 units/gram, previously filtered from the preservation solution and washed on the filter with deionized water, was added to 2 litres of a solution kept thermostatically at 60°C and having the following composition:
   30 mM Ara-U
   30 mM 2,6-diaminopurine (DAMP)
   30 mM potassium phosphate buffer, pH 7.
   After 2.5 hours at 60°C, the catalyst was discharged (and was made to react in a new bioconversion reaction) and the filtrate was cooled in order to recover the precipitate, constituted by crude Ara-DAMP, which was purified by crystallization from water.
   The bioconversion yield of the reaction was ≥ 70%. The purity of Ara-DAMP after crystallization, determined by HPLC analysis, was greater than 98%.
c) Preparation of β-D-arabinofuranosyl-2-fluoroadenine des-phosphate (fludarabine des-phosphate) 30 grams (moist weight) of immobilized catalyst with transglycosylation activity of about 13 units/gram, previously filtered from the preservation solution and washed on the filter with deionized water, was added to 800 ml of a solution containing 40% of dimethyl sulphoxide (DMSO) kept thermostatically at 60°C and having the following composition:
   75 mM Ara-U
   50 mM 2-fluoro-adenine
   30 mM potassium phosphate buffer, pH 7.
   After 4 hours at 60°C, the catalyst was discharged (and was made to react in a new bioconversion reaction) and the filtrate was cooled in order to recover the precipitate, constituted by crude fludarabine desphosphate, which was purified by crystallization from a DMSO/water mixture.
   The bioconversion yield of the reaction was ≥ 70%. The purity of fludarabine des-phosphate after crystallization, determined by HPLC analysis, was greater than 98%:
d) Preparation of 2'-deoxyadenosine
   1 gram (moist weight). of immobilized catalyst with transglycosylation activity of about 13 units/gram, previously filtered from the preservation solution and washed on the filter with deionized water, was added to 200 ml of a solution kept thermostatically at 60°C and having the following composition:
   75 mM 2'-deoxyuridine
   50 mM adenine
   30 mM phosphate buffer pH 7.

After 2 hours at 60°C the catalyst was discharged (and was made to react in a new bioconversion reaction) and the filtrate was cooled in order to recover the precipitate, constituted by 2'-deoxyadenosine, which was purified by precipitation from H₂O. The bioconversion yield of the reaction was ≥ 70%. The purity of the 2'-deoxyadenosine after crystallization, determined by HPLC analysis, was greater than 98%.
a) Preparation of 2'-deoxyguanosine
   5 grams (moist weight) of immobilized catalyst with transglycosylation activity of about 13 units/gram, previously filtered from the preservation solution and washed on the filter with deionized water, was added to 90 ml of a solution kept thermostatically at 60°C and having the following composition:
   60 mM 2'-deoxyuridine
   30 mM phosphate buffer pH 7.
   5 ml of an 0.8M solution of guanine in 30% NaOH was added very slowly (2.5 ml/hour), whilst the reaction mixture was kept at pH 7 by the continuous addition of 20% HCl. After 2.5 hours at 60°C, the catalyst was discharged (and was made to react in a new bioconversion reaction) and the filtrate was cooled to 50°C in order to eliminate the unreacted guanine by filtration. The filtrate was then cooled to 4°C in order to precipitate the 2' -deoxyguanosine which was purified by crystallization from H₂O. The bioconversion yield of the reaction was ≥ 70%. The purity of the 2'-deoxyguanosine after crystallization, determined by HPLC analysis was greater than 98%.
a) Preparation of (N'-β-D-ribofuranosyl)- 5-hydroxyimidazolo-4-carboxamide (mizoribine).
   5 grams (moist weight) of immobilized catalyst with transglycosylation activity of about 12 units/gram, previously filtered from the preservation solution and washed, on the filter, with deionized water, was added to 100 ml of a solution kept thermostatically at 60°C and having the following composition:
   225 mM uridine
   150 mM hydroxycarbamoyl-imidazole
   30 mM potassium phosphate buffer, pH 7.
   After 6 hours at 60°C the catalyst was discharged (and was made to react in a new bioconversion reaction), the filtrate was cooled, and the mizoribine was purified therefrom by chromatography in an ion-exchange column.
   The bioconversion yield of the reaction was ≥ 80%. The purity of mizoribine, determined by HPLC analysis, was greater than 98%.

a) Preparation of 2'-deoxyadenosine in a continuous reactor.

A preparation of immobilized catalyst with transglycosylation activity of about 12 units/gram was packed to a height of 4 cm in a column kept thermostatically at a temperature of 40°C and having a diameter of 2 cm, (column volume = 12.5 ml). 3 litres of a solution preheated to 40°C and containing 10 mM adenine, 15 mM 2'-deoxyuridine and 30 mM phosphate buffer, pH 7, was pumped through the column of catalyst by means of a pump with a calibrated flow of 2.5 ml/minute (flow rate = 1/5 of the column volume/minute). Aliquots of the eluate were collected after 30 and 60 minutes and then at 2-hourly intervals up to 20 hours and were analyzed by HPLC to determine the bioconversion yield which was within the range between 83% and 86% for all of the samples.

## Claims

1. Biocatalyst usable for transglycosylation reactions, containing a solid matrix functionalized with epoxy groups and the enzymes uridine phosphorylase and purine nucleoside phosphorylase, **characterized in that** the enzymes uridine phosphorylase and purine nucleoside phosphorylase are co-immobilized on the solid matrix by the formation of covalent bonds.

2. Biocatalyst according to Claim 1, **characterized in that** the covalent bonds are produced by reaction between the epoxy groups of the solid matrix and the amino groups of the enzymes.

3. Biocatalyst according to Claim 2, **characterized in that** the amino groups are amino groups of the lysine residues present on the polypeptide chains of the enzymes.

4. Biocatalyst according to Claims 1-3, **characterized in that** the solid matrix has a concentration of epoxy groups no less than 50 µmoles/gram of moist resin, preferably a concentration of about 100 µmoles/gram of moist resin.

5. Biocatalyst according to Claims 1-3, **characterized in that** the solid matrix is an epoxy resin.

6. Biocatalyst according to Claim 5, **characterized in that** the epoxy resin is selected from Epoxy-activated Sepharose ^{®} Fractogel^{®} TSK AF-Epoxy, Eupergit^{®} C, Eupergit^{®} C250L, Sepabeads^{®} EC-EP/M and Sepabeads^{®} EC-EP.

7. Biocatalyst according to Claim 5, **characterized in that** the epoxy resin has a particle size of between 200 and 600 microns.

8. Biocatalyst according to Claims 1-3, **characterized in that** the solid matrix is an agarose gel functionalized with epoxy groups, a silica gel functionalized with epoxy groups, a polymer based on methacrylamide-bisacrylamide functionalized with epoxy groups, or a polymethacrylate polymer functionalized with epoxy groups.

9. Biocatalyst according to Claims 1-8, **characterized in that** the enzymes UdP and PNP are of prokaryotic origin.

10. Biocatalyst according to Claims 1-8, **characterized in that** the enzymes UdP and PNP are produced by a recombinant method.

11. Biocatalyst according to Claim 10, **characterized in that** the enzymes UdP and PNP are contained in the crude soluble portion extracted from the recombinant cells or in partially purified or purified preparations derived from the crude soluble portion extracted from the recombinant cells.

12. Use of the biocatalyst according to Claims 1 to 11 for the production of nucleosides by an enzymatic transglycosylation reaction between a sugar-donor nucleoside and a heterocyclic sugar-acceptor base.

13. Use according to Claim 12, **characterized in that** the sugar of the donor nucleoside is β-D-ribose, 2'deoxy-β-D-ribose, 2'-3'-dideoxy-β-D-ribose, β-D-arabinose, α-L-xylose, or amino-derivatives or halo-derivatives thereof.

14. Use according to Claim 12, **characterized in that** the base of the donor nucleoside is uracil.

15. Use according to Claims 12 to 14, **characterized in that** the transglycosylation reaction is carried out in aqueous phase.

16. Use according to Claims 12 to 14, **characterized in that** the transglycosylation reaction is carried out in the presence of phosphate buffer.

17. Use according to Claims 12 to 14, **characterized in that** the transglycosylation reaction is carried out at a pH of between 6 and 8, preferably between 6.5 and 7.5.

18. Use according to Claims 12 to 14, **characterized in that** the transglycosylation reaction is carried out at temperatures of up to 75°C and preferably at temperatures of between 55 and 60°C.

19. Use according to Claims 12 to 18, **characterized in that**, upon completion of the reaction, the immobilized catalyst is separated from the reaction mixture by filtration, decantation, or centrifugation and is reused in successive transglycosylation reactions.

20. Use according to Claims 12 to 18, **characterized in that** the transglycosylation reaction is carried out in a continuous system in which the reaction is completed by passage through the biocatalyst which is kept in a column or in a thermostatically controlled continuous reactor.

21. Method for the preparation of a biocatalyst according to Claims 1 to 11, **characterized in that** the enzymes uridine phosphorylase and purine nucleoside phosphorylase are put in contact with the solid matrix, functionalized with epoxy groups, with stirring, at a pH of between 6 and 9, and at a temperature of between 10 and 50°C.

22. Method according to Claim 21, **characterized in that** the enzymes uridine phosphorylase and purine nucleoside phosphorylase are put separately in contact with two distinct fractions of the solid matrix which are then mixed together to give the biocatalyst.

23. Method according to Claims 21-22, **characterized in that** the enzymes uridine phosphorylase and purine nucleoside phosphorylase are used in uridine phosphorylase : purine nucleoside phosphorylase enzymatic-activity ratios of between 1 : 0.5 and 1 : 3, preferably in a ratio of about 1 : 1.

24. Method according to Claims 21-23, **characterized in that** (a) the biomass obtained by the fermentation of bacterial strains expressing the enzymes uridine phosphorylase and purine nucleoside phosphorylase are subjected to lysis and centrifuged to separate the crude soluble fractions, (b) the mixture of the crude soluble fractions thus obtained is put in contact with the solid matrix functionalized with epoxy groups without further purification steps.

25. Method according to Claim 24, **characterized in that** a single bacterial strain which simultaneously expresses the enzymes uridine phosphorylase and purine nucleoside phosphorylase is used, or two distinct bacterial strains one expressing the enzyme uridine phosphorylase and the other the enzyme purine nucleoside phosphorylase are used.

26. Method according to Claims 24-25, **characterized in that** the bacterial strains are recombinant.

27. Method according to Claims 24-26, **characterized in that**, prior to step (a), the biomass is subjected to a heat treatment at a temperature of 50-70°C, preferably at, 60°C.

28. Method according to Claim 27, **characterized in that** the heat treatment is performed for 20-40 minutes, preferably for 30 minutes.

29. A biocatalyst that can be produced by the method according to Claims 21-28.

## Patentansprüche

1. Biokatalysator, der für Transglycosylierungsreaktionen geeignet ist, enthaltend eine feste Matrix funktionalisiert mit Epoxygruppen und die Enzyme Uridinphosphorylase und Purinnucleosidphosphorylase, **dadurch gekennzeichnet, dass** die Enzyme Uridinphosphorylase und Purinnucleosidphosphorylase auf der festen Matrix durch die Bildung von kovalenten Bindungen co-immobilisiert sind.

2. Biokatalysator nach Anspruch 1, **dadurch gekennzeichnet, dass** die kovalenten Bindungen durch Reaktion zwischen den Epoxygruppen der festen Matrix und den Aminogruppen der Enzyme gebildet werden.

3. Biokatalysator nach Anspruch 2, **dadurch gekennzeichnet, dass** die Aminogruppen Aminogruppen der Lysinreste sind, die in den Polypeptidketten der Enzyme vorhanden sind.

4. Biokatalysator nach den Ansprüchen 1 bis 3, **dadurch gekennzeichnet, dass** die feste Matrix eine Konzentration von Epoxygruppen von nicht weniger als 50 µmol/g feuchtem Harz, bevorzugt eine Konzentration von etwa 100 µmol/g feuchtem Harz, aufweist.

5. Biokatalysator nach den Ansprüchen 1 bis 3, **dadurch gekennzeichnet, dass** die feste Matrix ein Epoxyharz ist.

6. Biokatalysator nach Anspruch 5, **dadurch gekennzeichnet, dass** das Epoxyharz ausgewählt ist aus Epoxy-aktivierter Sepharose^{®}, Fractogel^{®} TSK AF-Epoxy, Eupergit^{®} C, Eupergit^{®} C250L, Sepabeads^{®} EC-EP/M und Sepabeads^{®} EC-EP.

7. Biokatalysator nach Anspruch 5, **dadurch gekennzeichnet, dass** das Epoxyharz eine Teilchengröße zwischen 200 und 600 Mikron aufweist.

8. Biokatalysator nach den Ansprüchen 1 bis 3, **dadurch gekennzeichnet, dass** die feste Matrix ein mit Epoxygruppen funktiortalisiertes Agarosegel, ein mit Epoxygruppen funktionalisiertes Silicagel, ein mit Epoxygruppen funktionalisiertes Polymer auf Basis von Methacrylamid-Bisacrylamid oder ein mit Epoxygruppen funktionalisiertes Polymethacrylat-Polymer ist.

9. Biokatalysator nach den Ansprüchen 1 bis 8, **dadurch gekennzeichnet, dass** die Enzyme UdP und PNP prokaryotischen Ursprungs sind.

10. Biokatalysator nach den Ansprüchen 1 bis 8, **dadurch gekennzeichnet, dass** die Enzyme UdP und PNP durch ein rekombinantes Verfahren gebildet werden.

11. Biokatalysator nach Anspruch 10, **dadurch gekennzeichnet, dass** die Enzyme UdP und PNP in dem aus den rekombinanten Zellen extrahierten rohen löslichen Anteil oder in teilweise gereinigten oder gereinigten Zubereitungen, die von dem aus den rekombinanten Zellen extrahierten rohen löslichen Anteil abgeleitet sind, enthalten sind.

12. Verwendung des Biokatalysators nach den Ansprüchen 1 bis 11 für die Herstellung von Nucleosiden durch eine enzymatische Transglycosylierungsreaktion zwischen einem Zucker-Donornucleosid und einer heterocyclischen Zucker-Akzeptorbase.

13. Verwendung nach Anspruch 12, **dadurch gekennzeichnet, dass** der Zucker des Donornucleosids β-D-Ribose, 2'-Deoxy-β-D-ribose, 2'-3'-Dideoxy-β-D-ribose, β-D-Arabinose, α-L-Xylose oder Aminoderivate oder Halogenderivate davon ist.

14. Verwendung nach Anspruch 12, **dadurch gekennzeichnet, dass** die Base des Donornucleosids Uracil ist.

15. Verwendung nach den Ansprüchen 12 bis 14, **dadurch gekennzeichnet, dass** die Transglycosylierungsreaktion in wässriger Phase durchgeführt wird.

16. Verwendung nach den Ansprüchen 12 bis 14, **dadurch gekennzeichnet, dass** die Transglycosylierungsreaktion in Anwesenheit von Phosphatpuffer durchgeführt wird.

17. Verwendung nach den Ansprüchen 12 bis 14, **dadurch gekennzeichnet, dass** die Transglycosylierungsreaktion bei einem pH zwischen 6 und 8, bevorzugt zwischen 6,5 und 7,5, durchgeführt wird.

18. Verwendung nach den Ansprüchen 12 bis 14, **dadurch gekennzeichnet, dass** die Transglycosylierungsreaktion bei Temperaturen von bis zu 75°C und bevorzugt bei Temperaturen zwischen 55 und 60°C durchgeführt wird.

19. Verwendung nach den Ansprüchen 12 bis 18, **dadurch gekennzeichnet, dass** nach Vervollständigung der Reaktion der immobilisierte Katalysator aus der Reaktionsmischung durch Filtrieren, Dekantieren oder Zentrifugieren abgetrennt wird und in nachfolgenden Transglycosylierungsreaktionen wiederverwendet wird.

20. Verwendung nach den Ansprüchen 12 bis 18, **dadurch gekennzeichnet, dass** die Transglycosylierungsreaktion in einem kontinuierlichen System durchgeführt wird, in dem die Reaktion durch Hindurchleiten durch den Biokatalysator vervollständigt wird, der in einer Säule oder in einem thermostatisch gesteuerten kontinuierlichen Reaktor gehalten wird.

21. Verfahren zur Herstellung eines Biokatalysators nach den Ansprüchen 1 bis 11, **dadurch gekennzeichnet, dass** die Enzyme Uridinphosphorylase und Purinnucleosidphosphorylase unter Rühren bei einem pH zwischen 6 und 9 und bei einer Temperatur zwischen 10 und 50°C mit der mit Epoxygruppen funktionalisierten festen Matrix in Kontakt gebracht werden.

22. Verfahren nach Anspruch 21, **dadurch gekennzeichnet dass** die Enzyme Uridinphosphorylase und Purinnucleosidphosphorylase gesondert mit zwei verschiedenen Fraktionen der festen Matrix in Kontakt gebracht werden, die dann zusammengemischt werden, um den Biokatalysator zu ergeben.

23. Verfahren nach den Ansprüchen 21-22, **dadurch gekennzeichnet, dass** die Enzyme Uridinphosphorylase und Purinnucleosidphosphorylase in enzymatischen Aktivitätsverhältnissen Uridinphosphorylase : Purinnucleosidphosphorylase zwischen 1 : 0,5 und 1 : 3, bevorzugt in einem Verhältnis von etwa 1 : 1, verwendet werden.

24. Verfahren nach den Ansprüchen 21 bis 23, **dadurch gekennzeichnet, dass** (a) die Biomasse, die durch Fermentation von Bakterienstämmen, die die Enzyme Uridinphosphorylase und Purinnucleosidphosphorylase exprimieren, erhalten wird, einer Lysis unterworfen wird und zentrifugiert wird, um die löslichen Rohfraktionen zu trennen, (b) die Mischung der so erhaltenen löslichen Rohfraktionen ohne weitere Reinigungsschritte mit der mit Epoxygruppen funktionalisierten festen Matrix in Kontakt gebracht wird.

25. Verfahren nach Anspruch 24, **dadurch gekennzeichnet, dass** ein Bakterienstamm, der gleichzeitig die Enzyme Uridinphosphorylase und Purinnucleosidphosphorylase exprimiert, verwendet wird oder zwei verschiedene Bakterienstämme verwendet werden, wobei einer das Enzym Uridinphosphorylase exprimiert und der andere das Enzym Purinnucleosidphosphorylase.

26. Verfahren nach den Ansprüchen 24-25, **dadurch gekennzeichnet, dass** die Bakterienstämme rekombinant sind.

27. Verfahren nach den Ansprüchen 24 bis 26, **dadurch gekennzeichnet, dass** vor Schritt (a) die Biomasse einer Wärmebehandlung bei einer Temperatur von 50 bis 70°C, bevorzugt bei 60°C, unterworfen wird.

28. Verfahren nach Anspruch 27, **dadurch gekennzeichnet, dass** die Wärmebehandlung 20 bis 40 Minuten, bevorzugt 30 Minuten, durchgeführt wird.

29. Biokatalysator, der durch das Verfahren nach den Ansprüchen 21 bis 28 hergestellt werden kann.

## Revendications

1. Biocatalyseur utilisable pour des réactions de transglycosylation, contenant une matrice solide fonctionnalisée avec des groupes époxy et les enzymes uridine phosphorylase et purine nucléoside phosphorylase, **caractérisé en ce que** les enzymes uridine phosphorylase et purine nucléoside phosphorylase sont co-immobilisées sur la matrice solide par la formation de liaisons covalentes.

2. Biocatalyseur selon la revendication 1, **caractérisé en ce que** les liaisons covalentes sont produites par réaction entre les groupes époxy de la matrice solide et les groupes amino des enzymes.

3. Biocatalyseur selon la revendication 2, **caractérisé en ce que** les groupes amino sont les groupes amino des résidus lysine présents sur les chaînes polypeptidiques des enzymes.

4. Biocatalyseur selon les revendications 1 à 3, **caractérisé en ce que** la matrice solide a une concentration de groupes époxy non inférieure à 50 µmoles/gramme de résine humide, de préférence une concentration d'environ 100 µmoles/gramme de résine humide.

5. Biocatalyseur selon les revendications 1 à 3, **caractérisé en ce que** la matrice solide est une résine époxy.

6. Biocatalyseur selon la revendication 5, **caractérisé en ce que** la résine époxy est choisie parmi la Sepharose ® époxy-activée, le Fractogel® TSK AF-Epoxy, l'Eupergit® C, l'Eupergit® C250L, le Sepabeads® EC-EP/M et le Sepabeads® EC-EP.

7. Biocatalyseur selon la revendication 5, **caractérisé en ce que** la résine époxy a une granulométrie comprise entre 200 et 600 micromètres.

8. Biocatalyseur selon les revendications 1 à 3, **caractérisé en ce que** la matrice solide est un gel d'agarose fonctionnalisé avec des groupes époxy, un gel de silice fonctionnalisé avec des groupes époxy, un polymère à base de méthacrylamide-bisacrylamide fonctionnalisé avec des groupes époxy, ou un polymère de polyméthacrylate fonctionnalisé avec des groupes époxy.

9. Biocatalyseur selon les revendications 1 à 8, **caractérisé en ce que** les enzymes UdP et PNP sont d'origine procaryote.

10. Biocatalyseur selon les revendications 1 à 8, **caractérisé en ce que** les enzymes UdP et PNP sont produites par un procédé de recombinaison.

11. Biocatalyseur selon la revendication 10, **caractérisé en ce que** les enzymes UdP et PNP sont contenues dans la partie soluble brute extraite à partir des cellules recombinantes ou dans des préparations partiellement purifiées ou purifiées dérivant de la partie soluble brute extraite à partir des cellules recombinantes.

12. Utilisation du biocatalyseur selon les revendications 1 à 11, pour la production de nucléosides par une réaction de transglycosylation enzymatique entre un nucléoside donneur de sucre et une basse accepteuse de sucre hétérocyclique.

13. Utilisation selon la revendication 12, **caractérisée en ce que** le sucre du nucléoside donneur est le β-D-ribose, le 2'-désaxy-β-D-ribose, le 2',3'-didésoxy-β-D-ribose, le β-D-arabinose, l'α-L-xylose, ou leurs dérivés amino ou dérivés halogéno.

14. Utilisation selon la revendication 12, **caractérisée en ce que** la base du nucléoside donneur est l'uracile.

15. Utilisation selon les revendications 12 à 14, **caractérisée en ce que** la réaction de transglycosylation est mise en oeuvre en phase aqueuse.

16. Utilisation selon les revendications 12 à 14, **caractérisée en ce que** la réaction de transglycosylation est mise en oeuvre en présence de tampon phosphate.

17. Utilisation selon les revendications 12 à 14, **caractérisée en ce que** la réaction de transglycosylation est mise en oeuvre à un pH compris entre 6 et 8, de préférence entre 6,5 et 7,5.

18. Utilisation selon les revendications 12 à 14, **caractérisée en ce que** la réaction de transglycosylalion est mise en oeuvre à des températures allant jusqu'à 75°C et de préférence à des températures comprises entre 55 et 60°C.

19. Utilisation selon les revendications 12 à 18, **caractérisée en ce qu'**une fois la réaction terminée, le catalyseur immobilisé est séparé du mélange réactionnel par filtration, décantation ou centrifugation, et est réutilisé dans des réactions de transglycosylation successives.

20. Utilisation selon les revendications 12 à 18, **caractérisé en ce que** la réaction de transglycosylation est mise en oeuvre dans un système en continu dans lequel la réaction est achevée par passage au travers du biocatalyseur qui est maintenu dans une colonne ou dans un réacteur en continu thermostaté.

21. Procédé pour la préparation d'un biocatalyseur selon les revendications 1 à 11, **caractérisé en ce que** les enzymes uridine phosphorylase et purine nucléoside phosphorylase sont mises en contact avec la matrice solide, fonctionnalisée avec des groupes époxy, sous agitation, à un pH compris entre 6 et 9, et à une température comprise entre 10 et 50°C.

22. Procédé selon la revendication 21, **caractérisé en ce que** les enzymes uridine phosphorylase et purine nucléoside phosphorylase sont mises en contact séparément avec deux fractions distinctes de la matrice solide, qui sont ensuite mélangées ensemble pour donner le biocatalyseur.

23. Procédé selon les revendications 21 à 22, **caractérisé en ce que** les enzymes uridine phosphorylase et purine nucléoside phosphorylase sont utilisées à des rapports d'activité enzymatique d'uridine phosphorylase/purine nucléoside phosphorylase compris entre 1/0,5 et 1/3, de préférence à un rapport d'environ 1/1.

24. Procédé selon les revendications 21 à 23, **caractérisé en ce que** (a) la biomasse obtenue par la fermentation de souches bactériennes exprimant les enzymes uridine phosphorylase et purine nucléoside phosphorylase sont soumises à une lyse et centrifugées pour que les fractions solubles brutes soient séparées, (b) le mélange des fractions solubles brutes ainsi obtenues est mis en contact avec la matrice solide fonctionnalisée avec des groupes époxy sans autres étapes de purification,

25. Procédé selon la revendication 24, **caractérisé en ce qu'**une seule souche bactérienne qui exprime simultanément les enzymes uridine phosphorylase et purine nucléoside phosphorylase est utilisée, ou bien deux souches bactériennes distinctes, l'une exprimant l'enzyme uridine phosphorylase et l'autre l'enzyme purine nucléoside phosphorylase, sont utilisées.

26. Procédé selon les revendications 24 à 25, **caractérisé en ce que** les souches bactériennes sont recombinantes.

27. Procédé selon les revendications 24 à 26, **caractérisé en ce qu'**avant l'étape (a), la biomasse est soumise à un traitement thermique à une température de 50 à 70°C, de préférence à 60°C.

28. Procédé selon la revendication 27, **caractérisé en ce que** le traitement thermique est effectué pendant 20 à 40 minutes, de préférence pendant 30 minutes.

29. Biocatalyseur pouvant être produit par le procédé selon les revendications 21 à 28.
